# EUROPEAN PATENT APPLICATION

(11) **EP 1 977 678 A2**
(43) Date of publication of application: **08.10.2008**
(21) Application number: 08101573.7
(22) Date of filing: 13.02.2008
(51) Int. Cl.: A61B 1/005, A61B 1/267, A61B 1/07

(54) **Intubation assisting device**

(30) Priority: 27.02.2007 FI 20075144
(71) Applicant: Finnomedo OY, 70100 Kuopio (FI)
(72) Inventor: Haara, Junnu, 70110 Kuopio (FI); Kolehmainen, Kari, 70100 Kuopio (FI); Sundholm, Lars, 00100 Helsinki (FI)
(74) Representative: LEITZINGER OY

(57) **Abstract**

The invention relates to an intubation assisting device, comprising a flexible fiber package (32) and a control unit (31) with the fiber package connected thereto, whereby an endotracheal tube is intended to be positioned over the fiber package. The fiber package (32) features a light conductive element (17) for communicating light to a distal end (22) of the fiber package, a fiberoptic element (8) for supplying the control unit with an image of the object immediately ahead of the fiber package's distal end, and a pulling means (19) for flexing the fiber package in one specific direction. The control unit (31) includes elements (1-7) for presenting the user with an image supplied by the fiberoptic element (8), and actuating elements (20) for operating the pulling means (19). The fiber package (32) features two parallel light conductive elements (17), the element couple constituted thereby having one side thereof provided with said pulling means (19) and its opposite side with said fiberoptic element (8). The control unit features a camera element (4) provided with a screen element (2) for displaying an image.

## Description

The present invention relates to an intubation assisting device, comprising a flexible fiber package and a control unit with the fiber package connected thereto, whereby an endotracheal tube is intended to be positioned over the fiber package; said fiber package featuring a protective cover inside which is disposed a light conductive element for communicating light to a distal end of the fiber package, a fiberoptic element for supplying the control unit with an image of the object immediately ahead of the fiber package's distal end, and a pulling means for flexing the fiber package in a specific direction, said control unit including elements for presenting the user with an image supplied by the fiberoptic element, and actuating elements for operating the pulling means.

Intubation, which involves placing an endotracheal tube in the trachea of a patient for to ensure ventilation either before a surgical procedure or in emergency situations, is often a highly stressful, traumatic and occasionally unsuccessful procedure. This procedure often involves several possible complications. There is a risk of the endotracheal tube being inadvertently placed in the gullet and, in case this error passes unnoticed, the result could be injury or death of a patient. In addition, the endotracheal tube could inadvertently end up in a tracheal bifurcation leading to just one of the lungs, which may likewise, in an extreme case, result in the death of a patient. Moreover, intubation must be performed as quickly as possible, even in a matter of seconds, especially in accident situations on patients who do not breathe any more, since otherwise the result can be a serious injury or death of the patient.

The correct placement of an endotracheal tube requires visualization of the relevant anatomical features for ensuring that the tube has become properly inserted in the trachea instead of the gullet and also to a correct depth, i.e. an appropriate distance short of the point at which the trachea bifurcates for the lungs. This positioning of an endotracheal tube calls for depressing the tongue and a displacement of the epiglottis to enable guiding the tube into the trachea.

It is an object of the present invention to provide an improved intubation assisting device, which enables performing necessary procedures for passing an endotracheal tube into a correct opening and for positioning the same at a correct depth in a reliable fashion. In order to achieve this objective, an intubation assisting device according to the invention is characterized in that the fiber package is set in an arcuate shape substantially complementary to the usual curvature of an endotracheal tube, that the pulling means is disposed in the proximity of the protective cover's internal surface next to its inside curve and attached to the protective cover within a region of the fiber package's distal end, such that actuation of the pulling means results in a flexion of the fiber package in a specific direction in the fiber package's distal end region, and that the fiber package has its distal end provided with a sleeve element of transparent material.

The endotracheal tube, mountable over a fiber package (installation means) and insertable into the trachea by means of such package, can be provided with an air-filled bag or a cuff capable of securing the tube in place relative to the trachea. After the endotracheal tube has been inserted into its position in the trachea, the cuff is filled, the intubation assisting device is removed, and the endotracheal tube is connected to a bellows element or the like for delivering air into the trachea. The endotracheal tube may also come without a cuff, especially tubes used in operating rooms.

A few preferred embodiments for an intubation assisting device of the invention are presented in dependent claims 2-10.

Document WO 96/39917 describes one solution for delivering an endotracheal tube into the trachea. One problem in this solution is that the fiber package is provided with a number of bending elements and hence, for example in an emergency situation, putting the device to use requires an experienced user who has the skill to guide the endotracheal tube to a correct object by means of a control stick rotatable in various directions. Another problem with this device is that monitoring how the tip of a fiber tube progresses toward a target area is performed by means of an eyepiece, making the device even more difficult to control.

The solution according to the present invention enables providing an intubation assisting device of high operating reliability, enabling also a one-handed use of the device, whereby the other hand can be free e.g. for supporting the neck of a patient. In an assisting device according to the invention, pulling the trigger results in the fiber package flexing in one direction only and the remaining necessary guiding action is obtained by rotating the entire control unit around its longitudinal axis. A device of the invention is particularly useful in mobile units, such as ambulances and emergency vehicles, yet is well suited to hospital use as well.

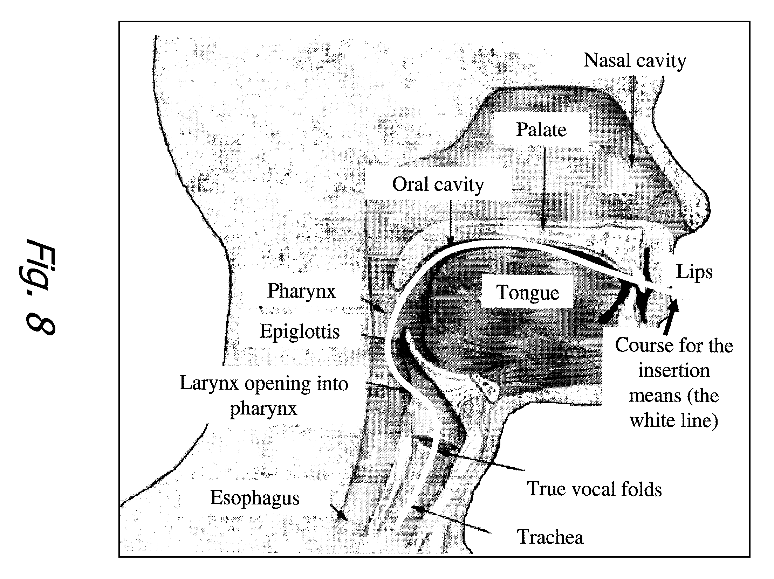

In terms of guiding a fiber package, the most important component is a protective cover for the fiber package. The protective cover, functioning as a shield for the fiber package, may consist of one or more components. The protective cover is an element, for example a tube, which is hollow in cross-section, extends from the end of a fiber package to the interior of a control unit, is curved consistently with the curvature of an endotracheal tube, and which functions as a shield for a fiberoptic element , a light conductive element and a pulling means extending within the same. In terms of bending resistance, elastic modulus, as well as cross-sectional shape, the protective cover material is selected in view of providing the fiber package with a sufficient strength for pushing it forward in a swollen pharynx yet enabling it to flex in compliance with human anatomical features. Fig. 8 indicates schematically the route of a fiber package in its delivery into the trachea. Providing the strength for pushing the end of a fiber package past obstacles is based on the aspect that, as the end of a fiber package meets with an obstacle, a propulsive force applied to the fiber package by the user compels the mid-section of a protective cover to flex against the palate, the propulsive force being transmitted to the fiber package's end by virtue of a supporting force produced by the palate. The patient-side end of a protective cover may consist of a short (about 30-50 mm in length) hollow element, for example a tube, having an elastic modulus which is substantially lower than that of the actual protective cover. Proximal to the short protective cover's inner curve, immediately behind the fiber package's end sleeve, is mounted a pulling means for example by means of a bonding agent. The pulling means extends in the proximity of the protective covers, i.e. closer to the fiber package's inner curve, to the interior of a control unit and attaches to an actuating means (trigger) used for flexing the fiber package. Pressing of the actuating means causes tensioning of the pulling means, resulting in a bending moment at a patient-side end of the fiber package, whereby this end of the fiber package flexes over a preferred distance towards the inner curve side. Releasing the actuating means results in the pulling wire slacking and the protective cover's end returning to a normal position as a result of the relaxation of a compressive stress on the inner curve side and a tensile stress on the outer curve side created in the flexing process (thus, the protective cover functions at the same time as a return spring).

In commercially available equipment (scopes, intubation accessories), the fiber package is generally either highly elastic or stiff. In the former case, it is best applicable to scoping e.g. the meandering intestines, yet cannot have sufficient strength applied thereto for by-passing obstacles (e.g. a swollen pharyngeal region). In the latter case, the device is in fact only applicable for a patient of exactly appropriate anatomy and even then may cause tissue damage. In an improved intubation assisting device of the present invention, the fiber package is sufficiently elastic for being guided, yet at the same time sufficiently stiff for pushing the endotracheal tube past obstacles and making the device as simple to use as possible. The user is only required to effect a control movement in just one axial direction for guiding the endotracheal tube correctly, i.e. a bending process of the fiber package by pressing the actuating means (trigger) and a returning process to the normal position by releasing the actuating means. Movements in other axial directions can be performed, if necessary, by turning the control unit.

The invention will now be described with reference to the accompanying drawings, in which:

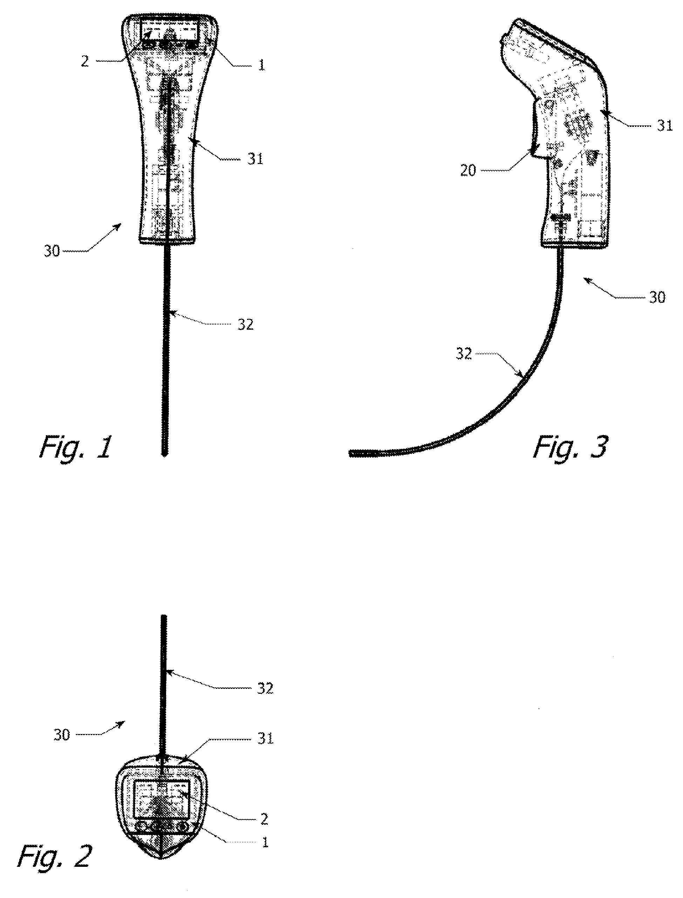

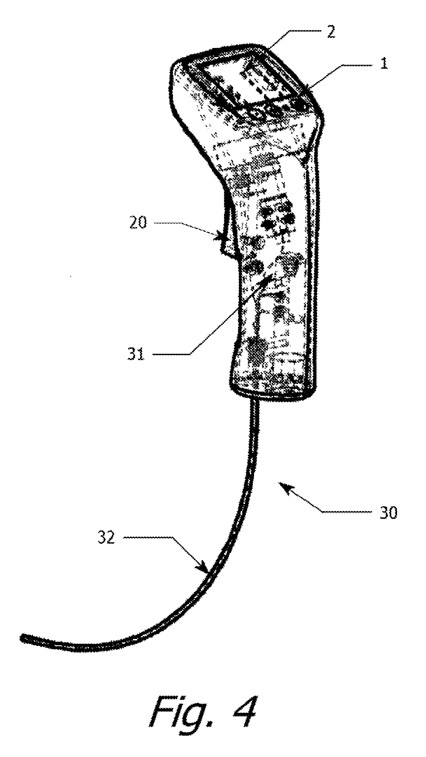

- Figs. 1-4: show an intubation assisting device of the present invention schematically in frontal, plan, elevation and perspective views, respectively,
- Fig. 5: shows an intubation assisting device of the invention, with the control unit depicted in a longitudinal section,
- Fig. 6: shows in more detail the distal end region of a fiber package in an enlarged scale,
- Fig. 7: shows in an enlarged perspective view of setting a light conductive element in alignment with a light source, and
- Fig. 8: shows schematically a path traveled by a fiber package in its passage to the trachea.

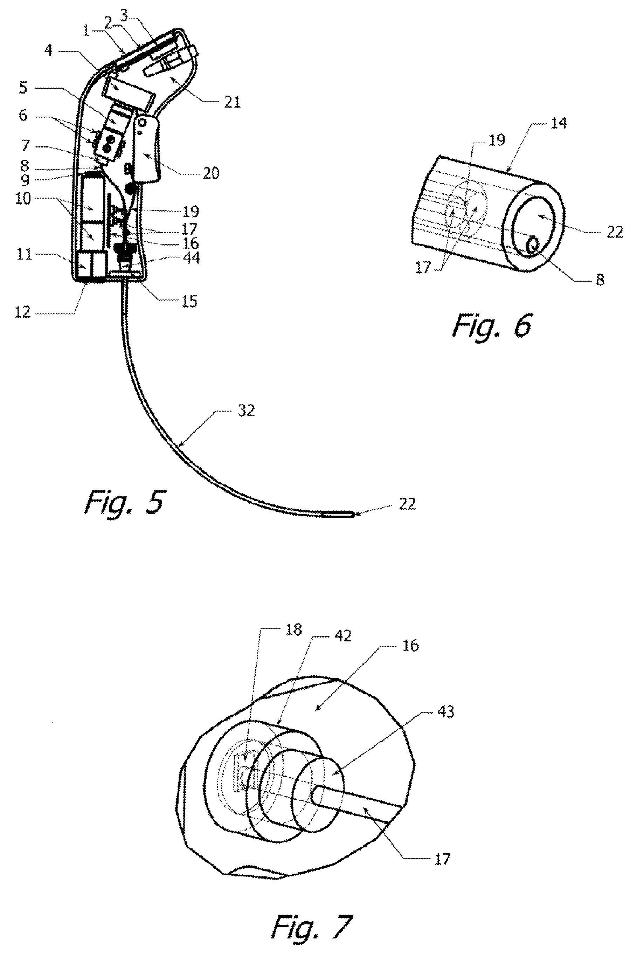

In reference to the figures, the intubation assisting device according to the invention, designated generally by reference numeral 30, comprises a control unit 31 and a flexible fiber package 32 which is preferably set in an arcuate shape as shown in the figures, complementary to the usual curvature of an endotracheal tube (not shown). The endotracheal tube is intended to be positioned over the fiber package 32. The control unit 31 includes a body component 21, which is provided with a trigger 21 having a pulling means 19 for the fiber package attached thereto for flexing the fiber package by pressing the trigger inward. The pulling means 19 is preferably made from a non-stretching material, which is attached to the trigger so as to establish a certain bias in the fiber package 32. In an exemplary embodiment, as shown in fig. 6, the fiber package consists of a light conductive element, which is made up for example of two parallel optical fibers 17, below which ( proximal to the outer curve) is disposed a fiberoptic element 8 and above which (proximal to the inner curve) is disposed the pulling means 19. The optical fibers 17 are coupled inside the body component 21 with a circuit board 16, which is provided, as a light source, preferably with surface-mounted LEDs 18 , whereby there is a LED associated with each optical fiber. Each optical fiber 17 is connected to its associated LED preferably by means of a sleeve joint of the invention, including an outer alignment sleeve 42 attached to the circuit board and enclosing a LED 18, and an inner optical-fiber fastening sleeve 43 insertable therein. The fastening sleeve features a hole accommodating an optical fiber in such a way that the optical fiber's proximal end becomes aligned with a LED 18 for communicating light from the LED via the optical fiber to an object present ahead of the fiber package's distal end. The light conductive element alignment and fastening sleeve can also be embodied in a single piece. The sleeve is centered over an illumination element in such a way that the light conductive element can be set in a precise registration against a light emitting surface of the illumination element for recovering as large a portion as possible of the luminosity. The sleeve over an illumination element is effective in absorbing the thermal energy emitted over a very small area by the illumination element and in releasing it, by virtue of an advantageous surface contour of the sleeve, into a vacant airspace present within the control unit, thus cooling the illumination element, whereby powerful localized heating is avoided and the LEDs provided with a longer service life. In addition, the device is preferably provided with adjustment means for regulating the power of light communicated to an object by means of a light conductive element.

## Claims

1. An intubation assisting device, comprising a flexible fiber package (32) and a control unit (31) with the fiber package connected thereto, whereby an endotracheal tube is intended to be positioned over the fiber package;
said fiber package (32) featuring a protective cover (14) inside which is disposed a light conductive element (17) for communicating light to a distal end (22) of the fiber package, a fiberoptic element (8) for supplying the control unit with an image of the object immediately ahead of the fiber package's distal end, and a pulling means (19) for flexing the fiber package in a specific direction,
said control unit (31) including elements (1-7) for presenting the user with an image supplied by the fiberoptic element (8), and actuating elements (20) for operating the pulling means (19),
**characterized in that** the fiber package (32) is set in an arcuate shape substantially complementary to the usual curvature of an endotracheal tube, that the pulling means (19) is disposed in the proximity of the protective cover's (14) internal surface next to its inside curve and attached to the protective cover within a region of the fiber package's (32) distal end (22), such that actuation of the pulling means results in a flexion of the fiber package in a specific direction in the fiber package's distal end region, and that the fiber package has its distal end provided with a sleeve element (22) of transparent material.

2. An intubation assisting device as set forth in claim 1, **characterized in that** the fiber package (32) features two parallel light conductive elements (17), the element couple constituted thereby having one side thereof, the side proximal to an inside curve of the fiber package, provided with said pulling means (19) and its opposite side with said fiberoptic element (8), and that the control unit features a camera element (4) provided with a screen element (2) for displaying an image.

3. An intubation assisting device as set forth in claim 2, **characterized in that** each light conductive element (17) consists of one or more optical fibers.

4. An intubation assisting device as set forth in any of claims 1-3,
**characterized in that** the control unit's camera is a CCD-cell equipped camera.

5. An intubation assisting device as set forth in any of claims 1-4,
**characterized in that** the control unit's screen element is an LCD display screen.

6. An intubation assisting device as set forth in any of the preceding claims,
**characterized in that** the fiberoptic element (8) has its end proximal to the control unit provided with a lens assembly (7), such that the fiberoptic element's position relative to the lens assembly is adjustable for adjusting the camera's focal length, and that the lens assembly (7) is disposed inside a coupling sleeve (5) interconnecting the camera (4) and the fiberoptic element's end proximal to the control unit, and that the coupling sleeve (5) is provided with adjustment/clamping means (6) enabling a rotation and/or an axial displacement of the lens assembly within the coupling sleeve for centering a screen-displayed image relative to the screen.

7. An intubation assisting device as set forth in any of the preceding claims,
**characterized in that** the control unit (31) features a battery/accumulator or battery/power pack accommodation space, one end of which is provided with contact elements (9) for a first terminal of the battery/accumulator or battery/power pack and the other end features a bushing element (11) with a threaded through-hole capable of having threaded therein a screw (12) coming to electrical contact with a second terminal of the battery/accumulator or battery/power pack set inside the battery enclosure, whereby the electrical current travels from the second terminal by way of the contact screw's (12) threads to the bushing element (11) and further to the point of use.

8. An intubation assisting device as set forth in any of the preceding claims,
**characterized in that** the light conductive elements are provided with light sources (18) present in a circuit board (16) set inside the control unit (31), each light conductive element being connected to its associated light source by way of a sleeve joint, including an outer alignment sleeve (42) attached to the circuit board and enclosing the light source, and an inner fastening sleeve (43) insertable therein, the latter being constructed with an accommodation hole for the light conductive element in such a way that the light conductive element's proximal end becomes aligned with the light source for communicating the light from the light source via the light conductive element (17) to an object present ahead of the fiber package's (32) distal end.

9. An intubation assisting device as set forth in any of the preceding claims,
**characterized in that** the device is capable of having attached thereto a sterilizing unit for after-use sterilization of the fiber package (32), said sterilizing unit comprising a tubular element, which is capable of being disposed over the fiber package, capable of being filled with a cleaning liquid, and which is closed at its distal end and attachable at its proximal end to the control unit (31) provided with a fastening element (44) receiving the proximal end of the tubular cleaning element in a sealing manner.

10. An intubation assisting device as set forth in claim 9, **characterized in that** the fastening element comprises a fastening cone (44), which is disposed inside the control unit (31), connected to the fiber package (32), and which positions itself inside the tubular cleaning element as the tubular element is pushed into the control unit.
